# EUROPÄISCHE PATENTANMELDUNG

(11) **EP 0 967 481 A2**
(43) Veröffentlichungstag der Anmeldung: **29.12.1999**
(21) Anmeldenummer: 99105364.6
(22) Anmeldetag: 16.03.1999
(51) Int. Cl.: G01N 33/00

(54) **Vorrichtung und Verfahren zur Messung von Abgaskomponenten**

(30) Priorität: 27.06.1998 DE 19828818
(71) Anmelder: Pierburg Aktiengesellschaft, 41460 Neuss (DE)
(72) Erfinder: Bornemann, Torsten, 41065 Mönchengladbach (DE)

(57) **Zusammenfassung**

Die Erfindung betrifft eine Vorrichtung und ein Verfahren zur Messung von Abgaskomponenten. Über eine beheizte Meßgaszuleitung wird zumindest einem Analysator Abgas zugeführt. Durch die besondere Anordnung des Leitungssystems sowie des Haupt- und Probenfördermittels, ist es auf besonders einfache Art und Weise möglich, Meßwerte zu ermitteln, die eine große Reproduzierbarkeit gewährleisten, wobei eine Kondensation von Komponenten im Abgas ausgeschlossen wird.

## Beschreibung

Die Erfindung betrifft eine Vorrichtung und ein Verfahren zur Messung von Abgaskomponenten gemäß den Oberbegriffen der Ansprüche 1 und 13.

Vorrichtungen und Verfahren zur Messung von Abgaskomponenten von Brennkraftmaschinen sind hinlänglich bekannt. Dabei wird Rohabgas oder verdünntes Abgas, das nachfolgend als Meßgas bezeichnet wird, einem oder mehreren Analysatoren zugeführt, um die Konzentration von Abgaskomponenten, wie zum Beispiel CO, CO2, NO oder Kohlenwasserstoffen zu messen. Um die Analysatoren in regelmäßigen Abständen zu kalibrieren, werden sogenannte Prüf-/Nullgase eingesetzt. Die Prüf-/Nullgase oder das Meßgas werden dabei durch ein Hauptfördermittel in einem Hauptgasstrom gefördert. Über eine Verzweigestelle wird dann ein Probengasstrom, der also entweder durch das Meßgas oder durch das Prüf-/Nullgas gebildet wird, vom Hauptgasstrom durch ein Probenfördermittel zum Analysator abgezweigt. Die Umstellung von Meßgas auf Prüf-/Nullgas erfolgt durch ein Umschaltventil.

Bei der Messung von Brennkraftmaschinenabgasen ergibt sich nun das Problem, daß insbesondere alle Aggregate der Vorrichtung zur Messung von Abgaskomponenten weitestgehend gegenüber Umwelteinflüssen, wie zum Beispiel Temperaturschwankungen, Schwingungen, etc. abgeschirmt sein sollten, um eine möglichst genaue und reproduzierbare Messung zu gewährleisten. Desweiteren muß die Kondensation von Komponenten, insbesondere Wasser, im Meßgas vermieden werden, da ansonsten das Meßergebnis verfälscht wird. Um die Bildung von Kondensat auszuschließen, muß das Meßgas dementsprechend hoch erhitzt werden. Mit der erhöhten Temperatur des Meßgases steigen jedoch der konstruktive Aufwand und damit die Kosten für das Leitungssystem und die zugehörigen Aggregate, insbesondere das Umachaltventil.

Der Erfindung liegt daher die Aufgabe zugrunde, eine Vorrichtung und ein Verfahren zur Messung von Abgaskomponenten zu schaffen, die die Messung von heißem bzw. beheiztem Meßgas, ohne die oben genannten Nachteile ermöglicht.

Diese Aufgabe wird durch die kennzeichnenden Merkmale der Ansprüche 1 und 13 gelöst.

Durch den beheizten Meßgasleitungsabschnitt im Leitungssystem wird eine Kondensation von beispielsweise Wasser im Meßgas verhindert. Das Prüf-/Nullgas enthält keine kondensierenden Komponenten und braucht deshalb nicht in dem Maße wie das Meßgas beheizt zu werden. Dadurch, daß das Prüf-/Nullgas zwischen der Verzweigestelle zur Bypasspumpe und dem Analysator zugeführt wird, kann das Umschaltventil entfallen, da bei Zuführung des Prüf-/Nullgases das gesamte Meßgas über das Hauptfördermittel geleitet wird.

Vorzugsweise weist die beheizte Meßgaszuleitung ein Filterelement auf, das im Meßgas enthaltene Festpartikel ausfiltert.

In einer besonderen Ausführungsform hat sich als Hauptfördermittel eine Bypasspumpe und als Probenfördermittel eine Vakuumpumpe als vorteilhaft erwiesen. Durch Durchflußbegrenzer im Meßgas-, Prüf-/Nullgas sowie Probengasleitungsabschnitt werden auf einfache Weise die Strömungsgeschwindigkeiten und die Drücke im Leitungssystem geregelt. Vorteilhafterweise sind die Durchflußbegrenzer als Düse oder Kapillare ausgeführt.

Um Druckschwankungen durch gezieltes Zuführen von Umgebungsluft auszugleichen, weist das Leitungasystem einen Druckregler auf, der stromaufwärts von der Bypasspumpe angeordnet sein kann. Um insbesondere den Druck im Probenleitungsabschnitt konstant zu halten, wird der Druckregler durch einen Drucksensor gesteuert, der vor dem Analysator angeordnet ist.

Um sogenannte "tote Äste" im Prüf-/Nullgasleitungsabschnitt und im Drucksensorleitungsabschnitt zu vermeiden, können diese Leitungsabschnitte direkt über Durchflußbegrenzer mit der Vakuumpumpe verbunden werden.

Zur Messung von Stickoxid weist der Analysator vorteilhafterweise eine Reaktionskammer auf, die über einen Durchflußbegrenzer in einem Reaktionsgasleitungsabschnitt mit einer Reaktionsgaszuleitung verbunden ist.

Um die Reproduzierbarkeit der Meßergebnisse noch weiter zu erhöhen, den Einfluß von Umgebungsparametern zu verringern und gleichzeitig schnelle Ansprechzeiten des Analysators zu erzeugen, ist es vorteilhaft, bei dem oben genannten Leitungssystem Leitungssystemkomponenten, wie zum Beispiel Leitungsabschnitte, Verzweigestellen, Durchflußbegrenzer, Sensoren, etc. in/an einem massiven, blockförmigen, wärmeleitfähigen Element anzuordnen, das ein Heizelement und einen Temperatursensor aufweist, und das an zumindest einer Außenseite Kopplungselemente für den/die Analysator(en), die jeweiligen Zuleitungen und Fördermittel aufweist. Temperaturschwankungen sowie Schwingungen können auf diese Weise keinen Einfluß mehr auf das Leitungssystem nehmen. Auch ist es möglich, die Temperatur des Leitungssystems auf eine einfache Weise zu steuern, was die Reproduzierbarkeit der Meßergebnisse erhöht. Weiterhin werden der Montageaufwand und damit auch die Kosten für das Leitungssystem verringert. Aufgrund der kurzen Leitungsabschnitte kann der Bauraum des Leitungssystems verringert werden und gleichzeitig schnelle Ansprechzeiten des Analysators realisiert werden. Zur Messung von Stickoxyden kann die Reaktionskammer in dem massiven, blockförmigen, wärmeleitfähigen Element angeordnet sein, wobei der Analysator beispielsweise durch eine Flanschverbindung direkt mit der Reaktionskammer verbunden werden kann.

Die Erfindung wird anhand eines in der Zeichnung dargestellten Ausführungsbeispiels näher erläutert.

Es zeigen:
- Fig. 1: einen Fließplan zur Messung von Meßgaskomponenten, und
- Fig. 2: eine perspektivische Ansicht des Leitungssystems in Form eines blockförmigen Elements,
- Fig. 3: eine Einzelansicht aus Fig. 2.

Über eine beheizte Meßgaszuleitung 1 wird Meßgas zur Messung der Meßgaskomponenten einem Leitungssystem 4 zugeführt. Das Meßgas durchläuft dabei außerdem ein Filterelement 9 zur Ausfilterung von Partikeln. Gefördert wird das Abgas durch eine Bypasspumpe 6. Desweiteren weist der Meßgasleitungsabschnitt einen Durchflußbegrenzer 12 auf. An der Verzweigestelle 5 wird ein Teil des Meßgasstromes durch eine Vakuumpumpe 7 als Probengas einem Analysator 3 zugeführt. Kurz vor dem Analysator ist in einem Probengasleitungsabschnitt 11 ein weiterer Durchflußbegrenzer 14 angeordnet.

Um den Druck im Probengasleitungsabschnitt 11 konstant zu halten, wird der Druck des Probengasstromes über eine Verzweigestelle 20 und einen Drucksensorleitungsabschnitt 17 einem Drucksensor 16 zugeführt. Dieser Drucksensor 16 ist über einen elektronischen Regelkreis mit einem Druckregler 15 verbunden, der den Druck im Hauptgasstrom regelt.

Zur Kalibrierung des Analysators 3 muß dem Analysator 3 in bestimmten Abständen ein Prüfgas bzw. Nullgas zugeführt werden. Dies geschieht über die Prüf-/Nullgaszuleitung 2 und den Prüf-/Nullgasleitungsabschnitt 10. Auch dieser Leitungsabschnitt weist einen Durchflußbegrenzer 13 auf. Das durch die Meßgaszuleitung 1 strömende Meßgas gelangt, die richtige Auslegung der Durchflußbegrenzer vorausgesetzt, nun nicht mehr zum Analysator 3. Auf diese Weise kann das bei den bekannten Analysevorrichtungen angeordnete Umschaltventil entfallen.

Unter Umständen kann sich das Problem stellen, daß der Drucksensorleitungsabschnitt 17 und der Prüf-/Nullgasleitungsabschnitt 10 keine Gasströme aufweisen, also wie "tote Äste" wirken. Die Ansprechzeiten des Analysators 3 können sich hierdurch erheblich verlängern. Um dies zu verhindern, werden die genannten Leitungsabschnitte über Durchflußbegrenzer 18 bzw. 19 direkt mit der Vakuumpumpe verbunden. Das hat zur Folge, daß diese Leitungsabschnitte gespült, d. h. kontinuierlich mit einer Gasströmung versehen werden.

Zur Messung von Stickoxyden ist desweiteren eine Reaktionskammer 29 an den Analysator 3 angeschlossen. Diese Reaktionskammer 29 ist über einen Durchflußbegrenzer 22, der in dem Reaktionsgasleitungsabschnitt 21 angeordnet ist, mit einer Reaktionsgaszuleitung 23, beispielsweise einem Ozonator, verbunden.

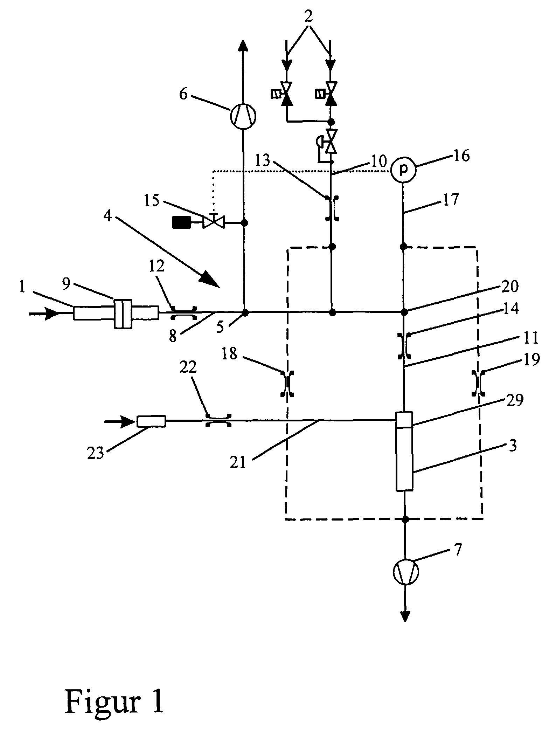

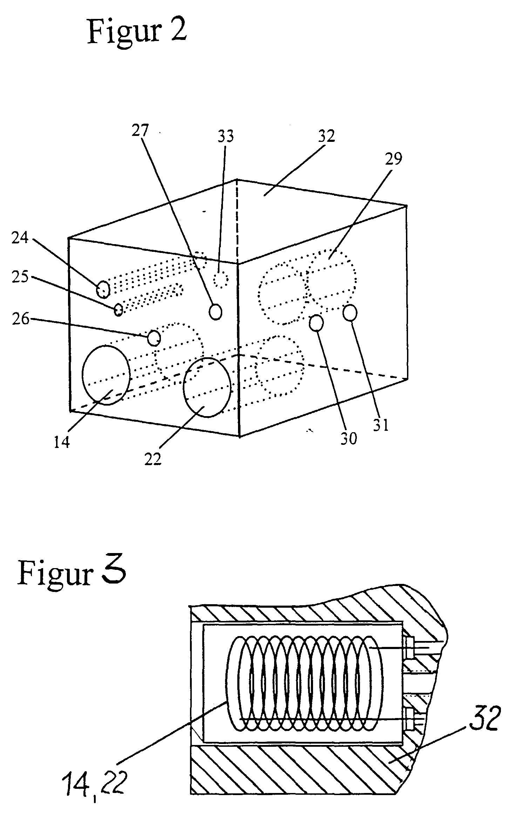

Fig. 2 zeigt nun das Leitungssystem 4 in Form eines blockförmigen Elements 32. Im vorliegenden Ausführungsbeispiel ist das blockförmige Element 32 ein massiver beheizter und isolierter Metallblock. Die Darstellung beschränkt sich zwecks Deutlichkeit auf die vorgesehenen Kopplungselemente und Aussparungen. Die verschiedenen Komponenten des Leitungssystems sind gemäß dem in Fig. 1 dargestellten Fließschema über Leitungen miteinander verbunden. Die Leitungswege lassen sich dabei auf einfache Weise z. B, durch Bohren oder andere Fertigungsverfahren herstellen.

Das blockförmige Element 32 weist Aussparungen auf, in die ein Heizelement 24, ein Temperatursensor 25 sowie beispielsweise die Kapillaren 14 und 22 einzusetzen sind, die in Fig. 3 als eingegossene Kappilare dargestellt ist. Desweiteren zeigt Fig. 2 die in dem Leitungssystem 4 angeordnete Reaktionskammer 29, an die der Analysator 3 anzuschließen ist. Über ein Meßgaskopplungselement 26, Prüf-/Nullgaskopplungselement 27, ein Reaktionsgaskopplungselement 28 sowie ein Drucksensorkopplungselement 33 sind die jeweiligen Zuleitungen anzuschließen. Die Bypasspumpe 6 und die Vakuumpumpe 7 sind über Kopplungselemente 30 bzw. 31 mit dem Leitungssystem 4 zu verbinden.

Die Kapillaren 14, 22 können dabei beispielsweise in Spiralform eingegossen werden und dann als zylinderförmiges Element in die Aussparungen des blockförmigen Elements eingesetzt werden.

Es wird darauf hingewiesen, daß das gezeigte Beispiel lediglich eine besondere Ausführungsform der Erfindung darstellt. Es ist zum Beispiel durchaus denkbar, daß mehrere Analysatoren auf die genannte Art und Weise im Leitungssystem 4 angeordnet werden. Auch hinsichtlich der Anordnung der Durchflußbegrenzer und des Druckreglers sind Varianten denkbar. Auch kann das blockförmige Element 32 bei konventionellen Vorrichtungen zur Messung von Abgaskomponenten, also Vorrichtungen, die ein Umschaltventil aufweisen, eingesetzt werden.

## Patentansprüche

1. Vorrichtung zur Messung von Abgaskomponenten mit
- einer Meßgaszuleitung (1),
- einer Prüf-/Nullgaszuleitung (2),
- zumindest einem Analysator (3),
- einem Leitungssystem (4) zur Zuführung eines Probengasstromes über eine Verzweigestelle (5) vom Hauptgasstrom zum Analysator (3), und
- ein Hauptfördermittel (6) und zumindest ein Probenfördermittel (7),
dadurch gekennzeichnet, daß
- die Meßgaszuleitung (1) beheizt ist, und
- das Prüf-/Nullgas einem Leitungsabschnitt zwischen der Verzweigestelle (5) und dem Analysator (3) zuführbar ist.

2. Vorrichtung nach Anspruch 1, dadurch gekennzeichnet, daß die Meßgaszuleitung (1) ein Filterelement (9) aufweist.

3. Vorrichtung nach Anspruch 1 oder 2, dadurch gekennzeichnet, daß das Hauptfördermittel (6) eine Bypasspumpe ist.

4. Vorrichtung nach einem der vorhergehenden Ansprüche, dadurch gekennzeichnet, daß das Probenfördermittel (7) eine Vakuumpumpe ist.

5. Vorrichtung nach einem der vorhergehenden Ansprüche, dadurch gekennzeichnet, daß ein Meßgasleitungsabschnitt (8), ein Prüf-/Nullgasleitungsabschnitt (10), sowie ein Probengasleitungsabschnitt (11) jeweils einen Durchflußbegrenzer (12, 13, 14) aufweisen.

6. Vorrichtung nach Anspruch 5, dadurch gekennzeichnet, daß die Durchflußbegrenzer (12, 13, 14) eine Düse oder eine Kapillare sind.

7. Vorrichtung nach einem der vorhergehenden Ansprüche, dadurch gekennzeichnet, daß das Leitungssystem (4) einen Druckregler (15) aufweist, der Druckschwankungen durch gezieltes Zuführen von Umgebungsluft ausgleicht.

8. Vorrichtung nach Anspruch 7, dadurch gekennzeichnet, daß vor dem Analysator ein Drucksensor (16) angeordnet ist, der den Druckregler (15) steuert.

9. Vorrichtung nach Anspruch 8, dadurch gekennzeichnet, daß der Prüf-/Nullgasleitungsabschnitt (10) und ein Drucksensorleitungsabschnitt (17) direkt über Durchflußbegrenzer (18, 19) mit der Vakuumpumpe (7) verbunden sind.

10. Vorrichtung nach einem der vorhergehenden Ansprüche, dadurch gekennzeichnet, daß stromaufwärts eine Reaktionskammer (29) an den Analysator angeschlossen ist, die über einen Durchflußbegrenzer (22) in einem Reaktionsgasleitungsabschnitt (21) mit einer Reaktionsgaszuleitung (23) verbunden ist.

11. Leitungssystem zur Verwendung in einer Vorrichtung nach einem der vorhergehenden Ansprüche, dadurch gekennzeichnet, daß Leitungssystemkomponenten, Leitungsabschnitte, Verzweigestellen, Durchflußbegrenzer und Sensoren, etc. in einem massiven, blockförmigen, wärmeleitfähigen Element (32) angeordnet sind, das ein Heizelement (24) und einen Temperatursensor (25) aufweist, und das zumindest an einer Außenseite Kopplungselemente für den/die Detektoren, die jeweiligen Zuleitungen und Fördermittel aufweist.

12. Leitungssystem nach Anspruch 11 in Verbindung mit Anspruch 10, dadurch gekennzeichnet, daß die Reaktionskammer (29) in dem massiven blockförmigen Element (32) angeordnet ist.

13. Verfahren zur Messung von Abgaskomponenten wobei zumindest einem Analysator (3) ein Probengasstrom über eine Verzweigestelle (5) eines Leitungssystems (4) zugeführt wird, dadurch gekennzeichnet, daß
- das Meßgas in der Meßgaszuleitung (1) erhitzt wird, und
- zu Kalibrierzwecken dem Analysator (3) Prüf-/Nullgas über einen Leitungsabschnitt zugeführt wird, der zwischen der Verzweigestelle (5) und dem Analysator (3) angeordnet ist.

14. Verfahren nach Anspruch 10, dadurch gekennzeichnet, daß dem Analysator (3) ein Drucksensor (16) vorgeschaltet wird, der einen Druckregler (15) steuert, durch den Druckschwankungen im Leitungssystem (4) durch Zuführen von Umgebungsluft ausgeglichen werden.

15. Verfahren nach Anspruch 11 oder 12, dadurch gekennzeichnet, daß ein Drucksensorleitungsabschnitt (17) und ein Prüf-/Nullgasleitungsabschnitt (10) über Durchflußbegrenzer (18, 19) direkt mit einem Probenfördermittel (7) verbunden werden.
